# EUROPEAN PATENT SPECIFICATION

(11) **EP 2 468 267 B1**
(45) Date of publication and mention of the grant of the patent: **08.11.2017**
(21) Application number: 11194806.3
(22) Date of filing: 21.12.2011
(51) Int. Cl.: A61K 9/20, A61K 9/28, A61K 31/395

(54) **Bilayer Combination Composition of Vildagliptin and Gliclazide**
Zweischichten-Kombinationszusammensetzung aus Vildgliptin und Gliclazid
Composition de combinaison bicouche de vildagliptine et de gliclazide

(30) Priority: 29.07.2011 TR 201107482; 21.12.2010 TR 201010683
(43) Date of publication of application: 27.06.2012
(73) Proprietor: Sanovel Ilaç Sanayi Ve Ticaret Anonim Sirketi, 34460 Sariyer/Istanbul (TR)
(72) Inventor: Cifter, Ümit, 34398 Istanbul (TR); Türkyilmaz, Ali, 34398 Istanbul (TR); Yelken, Gülay, 34398 Istanbul (TR); Saydam, Mehtap, 34398 Istanbul (TR)
(74) Representative: Sevinç, Erkan

(56) References cited:
- WO-A1-2008/028914
- WO-A2-2010/092163
- US-A1- 2007 172 525
- GARBER A J ET AL: "Effects of vildagliptin on glucose control in patients with type 2 diabetes inadequately controlled with a sulphonylurea", DIABETES, OBESITY AND METABOLISM, BLACKWELL SCIENCE, vol. 10, no. 11, 1 November 2008 (2008-11-01), pages 1047-1056, XP009149673, ISSN: 1462-8902, DOI: 10.1111/J.1463-1326.2008.00859.X
- DATABASE WPI Week 201127 Thomson Scientific, London, GB; AN 2011-A18673 XP002670676, & CN 101 897 696 A (SHENZHEN AUSA PHARMED CO LTD) 1 December 2010 (2010-12-01)

## Description

### Field of Invention

The present invention relates to formulations made with vildagliptin or a pharmaceutically acceptable salt of vildagliptin and gliclazide or a pharmaceutically acceptable salt of gliclazide. The present invention particularly relates to a bilayer combination composition made from vildagliptin and gliclazide.

### Background of Invention

Vildagliptin is a dipeptidyl dipeptidase-IV (DPP-IV) inhibitor developed for use in the treatment of type 2 diabetes (non-insulin dependent diabetes). Vildagliptin inhibits the degradation of the dipeptidyl dipeptidase-IV enzyme, thereby inhibiting the effects of incretin hormones, glucagon-like peptide-1 (GLP-1), and of glucose-dependent insulinotropic peptide (GIP). The chemical designation of vildagliptin is (S)-{[(3-hydroxyadamantan-1-yl)amino]acetyl}pyrrolidine-2-carbonitrile, with the chemical structure illustrated below in Formula 1.

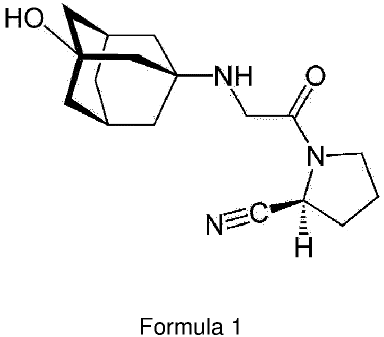

Vildagliptin is soluble in water and in organic polar solvents.

Vildagliptin is marketed under the trademark Galvus® in 50 mg dosage forms. It is used against diabetes mellitus, but particularly in treating type 2 diabetes.

There are various patents in the patent literature in relation to vildagliptin.

The patent application WO0034241 discloses vildagliptin or an acid addition salt thereof, as well as their use in diabetes mellitus and obesity.

The patent application WO2006078593 claims a direct-compression formulation of a DPP-IV inhibitor compound, and preferably of vildagliptin or an acid addition salt thereof.

The patent application WO2006135723 discloses a formulation, comprising vildagliptin as an active agent, as well as hydroxypropyl methyl cellulose, microcrystalline cellulose, and magnesium stearate.

Stability-related problems do occur in many active agents, including vildagliptin, under the influence of ambient and physical conditions. Vildagliptin is an active agent that is highly-susceptible to air and humidity. When vildagliptin is exposed to air and humidity, it degrades structurally and develops chemical behavioral changes. The stability of vildagliptin products developed is not at a desired level and the shelf life thereof is shortened. In addition, vildagliptin is reactive against the excipients employed in developing formulations containing the same. This fact causes impurities to occur in the formulation and leads to the inclusion of undesired components into the formulation.

Sulfonylurea is a name defining a group of orally administered anti-diabetic medicaments used in the treatment of type II diabetes mellitus. Diabetes mellitus is a disorder of the carbohydrate metabolism, in which insulin secretion is diminished, or which occurs due to a varying insulation secretion or decreased insulin activity or a combination of both factors. It is believed that sulfonylureas stimulate the insulin secretion from pancreatic islet cells in the mediation of receptors reported to be adenosine 5'-triphosphate-sensitive potassium channels. For this reason, sulfonylureas basically increase the endogenous insulin secretion so that an efficient control is provided in the blood sugar levels, not controllable via diets, of the diabetics and particularly of the type II diabetics.

Sulfonylureas are considered to be divided into two categories: first generation agents, e.g. tolbutamide, chlorpropamide, tolazamide, acetohexamide; and second generation agents, e.g. glyburide (glibenclamide), glipizide, gliclazide.

Gliclazide N-(4-methylbenzenesulfonyl)-N'-(3-azabicyclo[3.3.0]-oct-3-yl)urea), one type of second generation sulfonylureas, is an active agent which has antidiabetic properties at typical doses administered to humans (its chemical structure is illustrated with Formula 1).

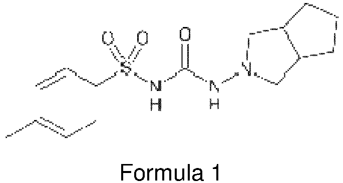

Gliclazide has been administered in the form of 80 mg conventional tablets until today. It is usually administrated one tablet twice a day, making a total of two tablets. It may be altered, however, to 1 to 4 tablets a day, as required by the severity of the diabetes case. A 30 mg modified-release dosage form is also available at the markets.

The formulation of gliclazide was first disclosed on 10.12.1996 with the patent document US 3,501,495 (SCIENCE UNION ET CIE. SOC.), disclosing the formula N-(4-methylbenzenesulphonyl)-N'-(3-azabicyclo[3.3.0]-oct-3-yl)urea), which has hypoglycemic properties, and is orally used in the treatment of diabetes mellitus.

Immediate-release formulations of sulfonylurea have been disclosed in many different patents in the past.

The patent US 4,696,815 discloses immediate-release tablets comprising sulfonylurea, formulated with an acidified and/or alkalized excipient and an inert polar solvent like polyethylene glycol. An analogous immediate-release formulation comprising an acidified and/or alkalized excipient, an inert polar solvent, and polyvinylpyrrolidone is also described in that patent.

The patent US 6,733,782 discloses a core tablet for the controlled-release of gliclazide, ensuring a sustained and constant release of the active agent by making use of hydroxypropylmethyl cellulose (HPMC) which is not influenced from the pH variations of the solubilizing medium following an oral administration. The main target of that invention is to obtain an oral dosage form, which can be administered once a day and provides extended release.

In the patent US 6,703,045 is disclosed an oral controlled-release system, which is beneficial in lowering serum glucose levels. That patent also describes a method of lowering serum glucose levels with an oral controlled-release dosage form comprising glipizide, and that once-a-day dosage form ensures to keep the medicament at a therapeutic level throughout the day.

The patent EP 1 741 435 A1 relates to an oral modified-release tablet composition comprising a pharmaceutically effective amount of a micronized active ingredient for lowering blood glucose level and a hydrophilic polymer, as well as to a method of preparing a novel composition for that modified-release oral tablet.

With the application WO 2006/061697 A1 is disclosed sustained-release pharmaceutical formulations suitable for once-a-day administration, as well as a process for preparing such formulations comprising sulfonylurea, polymer, disaccharide and/or monosaccharide (lactose, sucrose, maltose, galactose, trehalose, maltitol, dextrose or the mixtures thereof) exhibiting a drug release profile substantially independent of pH of the dissolution medium in pH range 4 to 8.

The gliclazide active agent is quite susceptible to humidity. Particularly if it is formulated with polymers having a hydrophilic structure, extreme stability problems are encountered. While it is desired to obtain a desired release profile with hydrophilic polymers, the affinity of this polymer to humidity brings about stability-related problems. This is unfavorable in terms of pharmaceutics. Therefore, a formulation is needed that would overcome this problem.

It is quite difficult to develop a formulation of vildagliptin and gliclazide with controlled release profiles of desired quality. Many excipients cause some problems, such as dissolution rate, compressibility, flowability, sticking to punches and other equipment during production, due to their higroscopicity, particle shape, size and density properties.

It is quite difficult to develop a controlled release formulation of vildagliptin and gliclazide which provides a bioavailable pharmaceutical composition according to the formulations currently used. The formulation of this invention represents a novel controlled release pharmaceutical composition of vildagliptin and gliclazide previously undisclosed in the prior art.

Making an assessment within the scope of said problems, it becomes obvious that a need exists for a stable combination composition of vildagliptin and gliclazide, which would provide a proper release profile.

### Object and Brief Description of Invention

The present invention provides a combination composition, eliminating all aforesaid problems and bringing additional advantages to the relevant prior art.

Accordingly, the main object of the present invention is to obtain a formulation, which is orally administered once or twice a day.

Another object of the present invention is to reduce the treatment onset time, with said formulation providing a 24-hour effect.

A further object of the present invention is to obtain a stable combination composition.

Another object of the present invention is to obtain a combination composition which provides the desired release profile.

A further object of the present invention is to prevent the production items from sticking to contact surfaces of machines and equipment.

A combination composition comprising two or more layers is developed to achieve all objects, referred to above and to emerge from the following detailed description.

In a preferred embodiment of the present invention, said novelty is characterized in that one layer of said composition comprises vildagliptin or a pharmaceutically acceptable salt or polymorph of vildagliptin, the other layer of said composition comprises gliclazide or a pharmaceutically acceptable salt or polymorph of gliclazide, and in that said composition comprises at least one further film layer, coating each of said layers individually or the combined surfaces of said layers, and containing a polyethylene glycol-polyvinyl alcohol copolymer or methacrylate polymer in an amount corresponding to 1 to 5% of the total composition weight.

A preferred embodiment of the present invention also contains one or more excipient(s).

In an embodiment of the present invention, one of said layers provides immediate release, whereas the other layer provides controlled release.

In a preferred embodiment of the present invention, the entirety of said layers provide immediate release.

In a preferred embodiment of the present invention, the entirety of said layers provide controlled release.

In a preferred embodiment of the present invention, said excipient(s) comprise(s) at least one or a mixture of controlled-release providing agents, fillers, binders, disintegrants, glidants, lubricants.

In a preferred embodiment of the present invention, said controlled-release providing agent comprises at least one or a properly-proportioned mixture of ethyl cellulose, polymethacrylate, polyethylene oxide, glyceryl behenate, hydroxypropyl methyl cellulose, hydroxypropyl cellulose, xanthan gum, stearoyl polyoxyl glycerides, and sodium alginate.

In a preferred embodiment of the present invention, said filler comprises at least one or a mixture of starch, mannitol, dibasic calcium phosphate, lactose, starch, calcium hydrogen phosphate dihydrate, dicalcium hydrogen phosphate anhydrate, calcium phosphate trihydrate, and glucose; but is preferably selected from mannitol and/or dibasic calcium phosphate.

In a preferred embodiment of the present invention, the proportion by weight of vildagliptin to mannitol is in the range of 0.01 to 12.

In a preferred embodiment of the present invention, the proportion by weight of gliclazide to dibasic calcium phosphate is in the range of 0.1 to 12.

In a preferred embodiment of the present invention, said binder is polyvinylpyrrolidone.

In a preferred embodiment of the present invention, said disintegrant is croscarmellose sodium.

In a preferred embodiment of the present invention, said glidant is at least one or a mixture of colloidal silicon dioxide, talk, aluminum silicate, magnesium silicate, with colloidal silicon dioxide being preferred.

In a preferred embodiment of the present invention, said lubricant is magnesium stearate.

In a preferred embodiment of the present invention, said croscarmellose sodium in extragranuler phase to total croscarmellose sodium is in the range of 0.1 to 0.8 preferably 0.2 to 0.7 and more preferably 0.25 to 0.6

Another preferred embodiment of the present invention comprises the following ingredients only:
a. immediate-release layer
   a. vildagliptin at 3.5 to 60% by weight,
   b. mannitol at 5 to 90% by weight,
   c. croscarmellose sodium at 6 to 25% by weight,
   d. polyvinylpyrrolidone at 0.1 to 25% by weight,
   e. colloidal silicon dioxide at 0.1 to 0.2% by weight,
   f. magnesium stearate at 0.25 to 2% by weight;
b. controlled-release layer
   a. gliclazide at 15 to 60% by weight,
   b. dibasic calcium phosphate at 5 to 90% by weight,
   c. ethyl cellulose/polymethacrylate at 0.5 to 40% by weight,
   d. colloidal silicon dioxide at 0.1 to 0.2% by weight,
   e. magnesium stearate at 0.25 to 2% by weight;
c. coating layer
   a. polyethylene glycol-polyvinyl alcohol copolymer or methacrylate polymer at 1 to 5% by weight.

A further preferred embodiment of the present invention provides a method for preparing a pharmaceutical formulation, this method comprising the steps of
a. sieving vildagliptin, croscarmellose sodium, and mannitol, and mixing in a high-shear granulator,
b. granulating the resulting mixture with an alcoholic/hydroalcoholic polyvinylpyrrolidone solution,
c. sieving the wet granules formed, drying the same, and sieving the dried granules again,
d. adding first colloidal silicon dioxide, and then magnesium stearate into the granules obtained,
e. sieving gliclazide and dibasic calcium phosphate, and mixing in a high-shear granulator,
f. granulating the resulting mixture with a hydroalcoholic ethyl cellulose/polymethylmethacrylate solution,
g. sieving the wet granules formed, drying the same, and sieving the dried granules again,
h. adding first colloidal silicon dioxide, and then magnesium stearate into the granules obtained,
i. compacting the controlled-release phase and the immediate-release phase to give a bilayer tablet,
j. coating the tablet with polyethylene glycol-polyvinyl alcohol copolymer or methacrylate polymer.

### Detailed Description of Invention

### Example 1

| **Immediate-release phase** | **weight %** |
|---|---|
| vildagliptin | 3.5 - 60 |
| mannitol | 5.0 - 90 |
| croscarmellose sodium | 6 - 25 |
| polyvinylpyrrolidone | 0.1 - 25 |
| colloidal silicon dioxide | 0.1 - 0.2 |
| magnesium stearate | 0.25 - 2.0 |

| **Controlled-release phase** | |
|---|---|
| gliclazide | 15 - 60 |
| Dibasic calcium phosphate | 5.0 - 90 |
| ethyl cellulose/polymethylmethacrylate | 0.5 - 40 |
| colloidal silicon dioxide | 0.1 - 0.2 |
| magnesium stearate | 0.25 - 2.0 |

| **Coating layer** | |
|---|---|
| polyethylene glycol-polyvinyl alcohol copolymer or methacrylate polymer | 1 - 5 |

### Production Method:

### Producing the Controlled-release phase

Gliclazide and dibasic calcium phosphate are sieved, and mixed in a high-shear granulator. Then, the resulting mixture is granulated with a hydroalcoholic ethyl cellulose/polymethylmethacrylate solution. Wet granules formed are sieved, then dried and the dried granules are sieved again. First colloidal silicon dioxide, and then magnesium stearate are added into the resulting granules to give the controlled-release phase.

### Producing the Immediate-release phase

Vildagliptin, croscarmellose sodium, and mannitol are sieved into a separate vessel and mixed in a high-shear granulator. Then, this mixture is granulated with an alcoholic/hydroalcoholic polyvinylpyrrolidone solution. Wet granules formed are sieved, then dried and the dried granules are sieved again. First colloidal silicon dioxide, and then magnesium stearate are added into the resulting granules to give the immediate-release phase. The controlled-release phase and the immediate-release phase are compacted to provide a bilayer tablet. The tablet obtained is coated with a humidity-barrier coating material, such as Opadry AMB/Kollicoat IR, to complete the process.

### Example 2

| **Controlled-release phase 1** | **weight %** |
|---|---|
| vildagliptin | 3.5 - 60 |
| mannitol | 5.0 - 90 |
| ethyl cellulose/polymethylmethacrylate | 0.5 - 40 |
| colloidal silicon dioxide | 0.1 - 0.2 |
| magnesium stearate | 0.25 - 2.0 |

| **Controlled-release phase 2** | |
|---|---|
| gliclazide | 15 - 60 |
| Dibasic calcium phosphate | 5.0 - 90 |
| ethyl cellulose/polymethylmethacrylate | 0.5 - 40 |
| colloidal silicon dioxide | 0.1 - 0.2 |
| magnesium stearate | 0.25 - 2.0 |
| polyethylene glycol-polyvinyl alcohol copolymer or methacrylate polymer | 1 - 5 |

### Production method

Vildagliptin, mannitol, and ethyl cellulose are sieved and mixed in a mixer. Thereafter, gliclazide, dibasic calcium phosphate, and ethyl cellulose are sieved and mixed in a separate mixer. Both phases are separately mixed first with colloidal silicon dioxide, and then with magnesium stearate. A compaction process is conducted in a bilayer tablet machine. The tablet obtained is coated with a humidity-barrier coating material, such as Opadry AMB/Kollicoat IR, to complete the process.

### Example 3

| **Controlled-release phase 1** | **weight %** |
|---|---|
| vildagliptin | 3.5 - 60 |
| stearyl polyoxyl glyceride | 0.5 - 50 |

| **Controlled-release phase 2** | |
|---|---|
| gliclazide | 15 - 60 |
| stearoyl polyoxyl glyceride | 0.5 - 90 |
| colloidal silicon dioxide | 0.1 - 0.2 |
| magnesium stearate | 0.25 - 2.0 |
| polyethylene glycol-polyvinyl alcohol copolymer or methacrylate polymer | 1 - 5 |

### Production Method:

Vildagliptin and gliclazide are separately melted with stearyl polyoxyl glyceride, and are then extruded, cold, and sieved. Both granules are mixed first with colloidal silicon dioxide, and then with magnesium stearate for a short time. A compaction process is conducted on the powder obtained in a bilayer tablet machine. The tablet obtained is coated with a humidity-barrier coating material, such as Opadry AMB/Kollicoat IR, to complete the process.

### Example 4

| **Immediate-release phase** | **weight %** |
|---|---|
| vildagliptin | 3.5 - 60 |
| mannitol | 5.0 - 90 |
| croscarmellose sodium (70% intragranular phase - 30% extragranular phase) | 6 - 25 |
| polyvinylpyrrolidone | 0.1 - 25 |
| colloidal silicon dioxide | 0.1 - 0.2 |
| magnesium stearate | 0.25 - 2.0 |

| **Controlled-release phase** | |
|---|---|
| gliclazide | 15 - 60 |
| Dibasic calcium phosphate | 5.0 - 90 |
| ethyl cellulose/polymethylmethacrylate | 0.5 - 40 |
| colloidal silicon dioxide | 0.1 - 0.2 |
| magnesium stearate | 0.25 - 2.0 |

| **Coating layer** | |
|---|---|
| polyethylene glycol-polyvinyl alcohol copolymer or methacrylate polymer | 1 - 5 |

### Producing the Immediate-release phase

Vildagliptin, 70% of croscarmellose sodium, and 50% of mannitol are sieved into a separate vessel and mixed in a high-shear granulator. Then, this mixture is granulated with an alcoholic/hydroalcoholic polyvinylpyrrolidone solution. Wet granules formed are sieved, then dried and the dried granules are sieved again. First 30% of croscarmellose sodium, 50% of Mannitol and colloidal silicon dioxide are sieved onto the granulated powder and mixed, and then magnesium stearate is added into the resulting granules to give the immediate-release phase.

Croscarmellose sodium also can use 50% of intragranular phase or extragranular phase. Mannitol can use 70% of intragranular phase and 30% extragranular phase.

### Producing the Controlled-release phase

Gliclazide and dibasic calcium phosphate are sieved, and mixed in a high-shear granulator. Then, the resulting mixture is granulated with a hydroalcoholic ethyl cellulose/ polymethylmethacrylate solution. Wet granules formed are sieved, then dried and the dried granules are sieved again. First colloidal silicon dioxide, and then magnesium stearate are added into the resulting granules to give the controlled-release phase. The controlled-release phase and the immediate-release phase are compacted to provide a bilayer tablet. The tablet obtained is coated with a humidity-barrier coating material, such as Opadry AMB/Kollicoat IR, to complete the process.

With this invention realized, a combination composition is obtained which has a surprising stability and release profile. One layer of said composition comprises vildagliptin or a pharmaceutically acceptable salt or polymorph of vildagliptin, and the other layer of said composition comprises gliclazide or a pharmaceutically acceptable salt or polymorph of gliclazide. At least one film coating layer is provided in the composition, wherein this layer provides a coating for each layer separately, or for the combined surfaces of said layers, and comprises polyethylene glycol-polyvinyl alcohol copolymer or methacrylate polymer in an amount corresponding to 1 to 5% of the total composition weight. Preferably mannitol and/or dibasic calcium phosphate, as the diluent, contribute(s) to the stability of the formulation.

With the present invention, combination formulations of gliclazide and vildagliptin are obtained, which have surprisingly good stability, solubility, and dissolution rate. The preferred coating layer materials are water soluble and quickly dissolves after swallowing. The seal coat provides a moisture barrier and friability of the tablet in order to minimize moisture and attritional effects.

As illustrated in example 4 some part of excipients is used intragranuler phase or extragranuler phase. This tablet therefore does not stick to equipment of machinery surface during the manufacture proses and shows desired release profiles and good compressibility, good flowability.

The formulation developed is used in the treatment of diabetes mellitus.

The term combination composition both includes multilayer formulations, and separate-use, fixed dose and kit formulations.

Although the obtained tablet is bilayered, it is alternatively possible to design said tablet with more layers as well. It is possible to provide one or more intermediate layer(s) between the layers containing the active agents in order to combine the layers, such intermediate layer(s) possibly including an active agent or not including an active agent, and providing immediate release or controlled release. Whilst both layers including active agents may provide controlled release or immediate release, at least one thereof may provide immediate release while the others provide controlled, prolonged, and retarded release.

It is further possible to use the following additional excipients in this formulation.

Fillers, but are not restricted to at least one or a mixture of starch, sugars, mannitol, sorbitol, sucrose, inorganic salts, calcium salts, polysaccharides, dextrose, dicalsium phosphate, sodium chloride, dextrates, lactitol, maltodextrin, sucrose-maltodextrin mixtures, xylitol, trehalose, etc..

Binders, but are not restricted to at least one or a mixture of polyvinylpyrrolidone, gelatin, sugars, glucose, natural gums, gums, synthetic celluloses, polymethacrylate, hydroxypropylmethyl cellulose, hydroxypropyl cellulose, carboxymethyl cellulose, methyl cellulose, and other cellulose derivatives, etc..

Glidants, but are not restricted to at least one or a mixture of colloidal silicon dioxide, talk, and aluminum silicate.

Lubricants, but are not restricted to at least one or a mixture of sodium stearyl fumarate, magnesium stearate, polyethylene glycol, stearic acid, metal stearates, boric acid, sodium chloride benzoate and acetate, sodium or magnesium lauryl sulfate, etc..

Disintegrants, but are not restricted to at least one or a mixture of sodium starch glycolate, croscarmellose sodium, crospovidone, sodium alginate, gums, starch, and magnesium aluminum silicate, etc..

Hot melts, but are not restricted to at least one or a mixture of Poloxamer 188 (polyoxyethylene-polyoxypropylene block copolymer), Gelucire 50/13 (stearyl macrogolglyceride), polyethylene glycol, povidone, Soluplus, cationic methacrylate, copovidone, methacrylic acid copolymers, cellulose acetate phthalate, acetyl monoglyceride, dibutyl tartrate, diethyl phthalate, dimethyl phthalate, glycerin, propylene glycol, triacetine, triacetine citrate and Tripropionin, etc..

Surface active agents, but are not restricted to at least one or a mixture of sodium lauryl sulfate, dioctyl sulfosuccinate, polysorbates and polyoxyethylene alkyl esters and ethers thereof, glyceryl monolaurate saponins, sorbitan laurate, sodium lauryl sulfate, and magnesium lauryl sulfate, etc..

Coating agents, but are not restricted to hydroxypropyl methyl cellulose, polyethylene glycol, polyvinylpyrrolidone, polyvinylpyrrolidone-vinyl acetate copolymer (PVP-VA), polyvinyl alcohol and other polymers, and all kinds of OpadryTM, as well as pigments, dyes, titanium dioxide and iron oxide, talk, etc..

## Claims

1. A combination composition comprising two or more layers, **characterized in that**
one layer of said composition comprises vildagliptin or a pharmaceutically acceptable salt or polymorph of vildagliptin,
the other layer of said composition comprises gliclazide or a pharmaceutically acceptable salt or polymorph of gliclazide,
said composition comprises at least one further film coating layer, coating each said layer separately, or the combined surfaces of said layers, and comprising polyethylene glycol-polyvinyl alcohol copolymer or methacrylate polymer in an amount corresponding to 1 to 5% of the total composition weight.

2. The combination composition according to Claim 1, further comprising at least one or more excipients.

3. The combination composition according to any of the preceding claims, wherein one of said layers is an immediate-release layer, and the other layer is a controlled-release layer.

4. The combination composition according to any of the preceding claims, wherein the entirety of said layers are immediate-release layers.

5. The combination composition according to any of the preceding claims, wherein the entirety of said layers are controlled-release layers.

6. The combination composition according to any of the preceding claims, wherein said excipients comprise at least one or a mixture of controlled-release providing agents, fillers, binders, disintegrants, glidants, and lubricants.

7. The combination composition according to any of the preceding claims, wherein said controlled-release providing agent is at least one or a mixture of ethyl cellulose, polymethacrylate, polyethylene oxide, glyceryl behenate, hydroxypropyl methyl cellulose, hydroxypropyl cellulose, xanthan gum, stearoyl polyoxyl glycerides, and sodium alginate.

8. The combination composition according to any of the preceding claims, wherein said filler is at least one or a mixture of starch, mannitol, dibasic calcium phosphate, lactose, starch, calcium hydrogen phosphate dihydrate, dicalcium hydrogen phosphate anhydrate, calcium phosphate trihydrate, and glucose; with mannitol and/or dibasic calcium phosphate being preferred.

9. The combination composition according to any of the preceding claims, wherein the proportion by weight of vildagliptin to mannitol is in the range of 0.01 to 12.

10. The combination composition according to any of the preceding claims, wherein the proportion by weight of gliclazide to dibasic calcium phosphate is in the range of 0.1 to 12.

11. The combination composition according to any of the preceding claims, wherein said binder is polyvinylpyrrolidone.

12. The combination composition according to any of the preceding claims, wherein said disintegrant is croscarmellose sodium.

13. The combination composition according to any of the preceding claims, wherein said glidant is at least one or a mixture of colloidal silicon dioxide, talk, aluminum silicate, and magnesium silicate; with colloidal silicon dioxide being preferred.

14. The combination composition according to any of the preceding claims, wherein said lubricant is magnesium stearate.

15. The combination composition according to any of the preceding claims, wherein said croscarmellose sodium in extragranular phase to total croscarmellose sodium is in the range of 0.1 to 0.8 preferably 0.2 to 0.7 and more preferably 0.25 to 0.6

16. The combination composition according to any of the preceding claims, comprising the following ingredients only:
a. immediate-release layer
a. vildagliptin at 3.5 to 60% by weight,
b. mannitol at 5 to 90% by weight,
c. croscarmellose sodium at 6 to 25% by weight,
d. polyvinylpyrrolidone at 0.1 to 25% by weight,
e. colloidal silicon dioxide at 0.1 to 0.2% by weight,
f. magnesium stearate at 0.25 to 2% by weight;
b. controlled-release layer
a. gliclazide at 15 to 60% by weight,
b. dibasic calcium phosphate at 5 to 90% by weight,
c. ethyl cellulose/polymethacrylate at 0.5 to 40% by weight,
d. colloidal silicon dioxide at 0.1 to 0.2% by weight,
e. magnesium stearate at 0.25 to 2% by weight;
c. coating layer
a. polyethylene glycol-polyvinyl alcohol copolymer or methacrylate polymer at 1 to 5% by weight.

17. A method for preparing a pharmaceutical formulation according to any of the preceding claims, comprising the steps of
a. sieving vildagliptin, croscarmellose sodium, and mannitol, and mixing in a high-shear granulator,
b. granulating the resulting mixture with an alcoholic/hydroalcoholic polyvinylpyrrolidone solution,
c. sieving the wet granules formed, drying the same, and sieving the dried granules again,
d. adding first colloidal silicon dioxide, and then magnesium stearate into the granules obtained,
e. sieving gliclazide and dibasic calcium phosphate, and mixing in a high-shear granulator,
f. granulating the resulting mixture with a hydroalcoholic ethyl cellulose/polymethylmethacrylate solution,
g. sieving the wet granules formed, drying the same, and sieving the dried granules again,
h. adding first colloidal silicon dioxide, and then magnesium stearate into the granules obtained,
i. compacting the controlled-release phase and the immediate-release phase to provide a bilayer tablet,
j. coating the tablet with polyethylene glycol-polyvinyl alcohol copolymer or methacrylate polymer.

18. The combination composition according to any of the preceding claims for preventing or treating diabetes mellitus in mammalians and particularly in humans.

## Patentansprüche

1. Kombinationszusammensetzung, umfassend zwei oder mehrere Schichten, **dadurch gekennzeichnet, dass**
eine Schicht der Zusammensetzung Vildagliptin oder ein pharmazeutisch annehmbares Salz oder Polymorph von Vildagliptin umfasst,
die andere Schicht der Zusammensetzung Gliclazid oder ein pharmazeutisch annehmbares Salz oder Polymorph von Gliclazid umfasst,
wobei die Zusammensetzung mindestens eine weitere Filmbeschichtungsschicht umfasst, wobei jeder Schicht getrennt aufgeschichtet wird, oder die vereinigten Oberflächen der Schichten, und umfassend Polyethylenglycol-Polyvinylalkohol-Copolymer oder Methacrylat-Polymer in einer Menge entsprechend 1 bis 5 % des Gewichts der gesamten Zusammensetzung.

2. Kombinationszusammensetzung nach Anspruch 1, weiterhin umfassend mindestens einen oder mehrere Hilfsstoffe.

3. Kombinationszusammensetzung nach irgendeinem der vorhergehenden Ansprüche, worin eine der Schichten eine sofort freisetzende Schicht ist und die andere Schicht ist eine Schicht mit gesteuerter Freisetzung.

4. Kombinationszusammensetzung nach irgendeinem der vorhergehenden Ansprüche, worin die Gesamtheit der Schichten sofort freisetzende Schichten sind.

5. Kombinationszusammensetzung nach irgendeinem der vorhergehenden Ansprüche, worin die Gesamtheit der Schichten Schichten mit gesteuerter Freisetzung sind.

6. Kombinationszusammensetzung nach irgendeinem der vorhergehenden Ansprüche, worin die Hilfsstoffe mindestens einen aus oder eine Mischung aus Mitteln, die die gesteuerte Freisetzung bereitstellen, Füllstoffen, Bindemitteln, Desintegrationsstoffen, Gleitmitteln und Schmiermitteln umfasst.

7. Kombinationszusammensetzung nach irgendeinem der vorhergehenden Ansprüche, worin das die gesteuerte Freisetzung bereitstellende Mittel mindestens eines ist aus oder eine Mischung aus Ethylcellulose, Polymethacrylat, Polyethylenoxid, Glycerylbehenat, Hydroxypropylmethylcellulose, Hydroxypropylcellulose, Xanthangummi, Stearoylpolyoxylglyceriden und Sodiumalginat.

8. Kombinationszusammensetzung nach irgendeinem der vorhergehenden Ansprüche, worin der Füllstoff mindestens eines aus oder eine Mischung aus Stärke, Mannitol, dibasischem Calciumphosphat, Lactose, Stärke, Caliumhydrogenphosphatdihydrat, Dicalciumhydrogenphosphatanhydrat, Calciumphosphattrihydrat und Glucose ist; wobei Mannitol und/oder dibasisches Calciumphosphat bevorzugt wird.

9. Kombinationszusammensetzung nach irgendeinem der vorhergehenden Ansprüche, worin der gewichtsmäßige Anteil von Vildagliptin zu Mannitol in dem Bereich von 0,01 bis 12 ist.

10. Kombinationszusammensetzung nach irgendeinem der vorhergehenden Ansprüche, worin der gewichtsmäßige Anteil von Gliclazid zu dibasischem Calciumphosphat in dem Bereich von 0,1 bis 12 ist.

11. Kombinationszusammensetzung nach irgendeinem der vorhergehenden Ansprüche, worin das Bindemittel Polyvinylpyrrolidon ist.

12. Kombinationszusammensetzung nach irgendeinem der vorhergehenden Ansprüche, worin das Desintegrationsmittel Croscarmellose-Natrium ist.

13. Kombinationszusammensetzung nach irgendeinem der vorhergehenden Ansprüche, worin das Gleitmittel mindestens eines aus oder eine Mischung aus kolloidalem Siliziumdioxid, Talk, Aluminiumsilicat und Magnesiumsilicat ist; wobei kolloidales Siliziumdioxid bevorzugt wird.

14. Kombinationszusammensetzung nach irgendeinem der vorhergehenden Ansprüche, worin das Schmiermittel Magnesiumstearat ist.

15. Kombinationszusammensetzung nach irgendeinem der vorhergehenden Ansprüche, worin das Croscarmellose-Natrium in extragranularer Phase zu Gesamt-Croscarmellose-Natrium in dem Bereich von 0,1 bis 0,8 ist, vorzugsweise 0,2 bis 0,7 und mehr bevorzugt 0,25 bis 0,6.

16. Kombinationszusammensetzung nach irgendeinem der vorhergehenden Ansprüche, umfassend nur die folgenden Bestandteile:
a. sofort freisetzende Schicht
a. Vildagliptin zu 3,5 bis 60 Gew.-%,
b. Mannitol zu 5 bis 90 Gew.-%,
c. Croscarmellose-Natrium zu 6 bis 25 Gew.-%,
d. Polyvinylpyrrolidon zu 0,1 bis 25 Gew.-%,
e. kolloidales Siliziumdioxid zu 0,1 bis 0,2 Gew.-%,
f. Magnesiumstearat zu 0,25 bis 2 Gew.-%;
b. Schicht mit gesteuerter Freisetzung
a. Gliclazid zu 15 bis 60 Gew.-%,
b. dibasisches Calciumphosphat zu 5 bis 90 Gew.-%,
c. Ethylcellulose/Polymethacrylat zu 0,5 bis 40 Gew.-%,
d. kolloidales Siliziumdioxid zu 0,1 bis 0,2 Gew.-%,
e. Magnesiumstearat zu 0,25 bis 2 Gew.-%;
c. Beschichtungsschicht
a. Polyethylenglycol-Polyvinylalkohol-Copolymer oder Methacrylat-Polymer zu 1 bis 5 Gew.-%.

17. Verfahren zur Herstellung einer pharmazeutischen Formulierung nach irgendeinem der vorhergehenden Ansprüche, umfassend die Schritte von
a. Sieben von Vildagliptin, Croscarmellose-Natrium und Mannitol und Vermischen in einem Hochscher-Granulator,
b. Granulieren der erhaltenen Mischung mit einer alkoholischen/hydroalkoholischen Polyvinylpyrrolidon-Lösung,
c. Sieben der gebildeten feuchten Granula, Trocknung derselben und erneutes Sieben der getrockneten Granula,
d. Zugabe von zunächst kolloidalem Siliziumdioxid und dann Magnesiumstearat in die erhaltenen Granula,
e. Sieben von Gliclazid und dibasischen Calciumphosphat und Vermischen in einem Hochscher-Granulator,
f. Granulieren der erhaltenen Mischung mit einer hydroalkoholischen Ethylcellulose/Polymethylmethacrylat-Lösung,
g. Sieben der gebildeten feuchten Granula, Trocknung derselben und erneutes Sieben der getrockneten Granula,
h. Zugabe von zunächst kolloidalem Siliziumdioxid und dann Magnesiumstearat in die erhaltenen Granula,
i. Kompaktierung der gesteuert freisetzenden Phase und der sofort freisetzenden Phase, um eine Zweischichtentablette zu erhalten,
j. Beschichtung der Tablette mit Polyethylenglycol-Polyvinylalkohol-Copolymer oder Methacrylat-Polymer.

18. Kombinationszusammensetzung nach irgendeinem der vorhergehenden Ansprüche zur Prävention oder Behandlung von Diabetes mellitus in Säugetieren, und insbesondere in Menschen.

## Revendications

1. Composition de combinaison comprenant au moins deux couches, **caractérisée par le fait que** :
une couche de ladite composition comprend de la vildagliptine ou un sel ou polymorphe pharmaceutiquement acceptable de vildagliptine,
l'autre couche de ladite composition comprend du gliclazide ou un sel ou polymorphe pharmaceutiquement acceptable de gliclazide,
ladite composition comprend au moins une autre couche de revêtement en film, revêtant chaque couche précitée séparément, ou les surfaces combinées desdites couches, et comprenant un copolymère polyéthylène glycol-alcool polyvinylique ou un polymère de méthacrylate dans une quantité correspondant à 1 à 5 % du poids total de composition.

2. Composition de combinaison selon la revendication 1, comprenant en outre au moins un ou plusieurs excipients.

3. Composition de combinaison selon l'une quelconque des revendications précédentes, dans laquelle l'une desdites couches est une couche à libération immédiate, et l'autre couche est une couche à libération contrôlée.

4. Composition de combinaison selon l'une quelconque des revendications précédentes, dans laquelle lesdites couches sont toutes des couches à libération immédiate.

5. Composition de combinaison selon l'une quelconque des revendications précédentes, dans laquelle lesdites couches sont toutes des couches à libération contrôlée.

6. Composition de combinaison selon l'une quelconque des revendications précédentes, dans laquelle lesdits excipients comprennent au moins l'un ou un mélange d'agents assurant une libération contrôlée, de charges, de liants, de désintégrants, d'agents de glissement et de lubrifiants.

7. Composition de combinaison selon l'une quelconque des revendications précédentes, dans laquelle ledit agent assurant une libération contrôlée est au moins l'un ou un mélange d'éthyl cellulose, de polyméthacrylate, de poly(oxyde d'éthylène), de béhénate de glycéryle, d'hydroxypropyl méthyl cellulose, d'hydroxypropyl cellulose, de gomme de xanthane, de stéaroyl polyoxyl glycérides et d'alginate de sodium.

8. Composition de combinaison selon l'une quelconque des revendications précédentes, dans laquelle ladite charge est au moins l'un ou un mélange d'amidon, de mannitol, de phosphate de calcium dibasique, de lactose, d'amidon, d'hydrogéno phosphate de calcium dihydraté, d'anhydrate hydrogéno phosphate dicalcique, de phosphate de calcium trihydraté et de glucose, le mannitol et/ou le phosphate de calcium dibasique étant préférés.

9. Composition de combinaison selon l'une quelconque des revendications précédentes, dans laquelle la proportion en poids de vildagliptine au mannitol se situe dans la plage de 0,01 à 12.

10. Composition de combinaison selon l'une quelconque des revendications précédentes, dans laquelle la proportion en poids de gliclazide au phosphate de calcium dibasique se situe dans la plage de 0,1 à 12.

11. Composition de combinaison selon l'une quelconque des revendications précédentes, dans laquelle ledit liant est la polyvinylpyrrolidone.

12. Composition de combinaison selon l'une quelconque des revendications précédentes, dans laquelle ledit désintégrant est la croscarmellose sodique.

13. Composition de combinaison selon l'une quelconque des revendications précédentes, dans laquelle ledit agent de glissement est au moins l'un ou un mélange de dioxyde de silicium colloïdal, de talc, de silicate d'aluminium et de silicate de magnésium, le dioxyde de silicium colloïdal étant préféré.

14. Composition de combinaison selon l'une quelconque des revendications précédentes, dans laquelle ledit lubrifiant est le stéarate de magnésium.

15. Composition de combinaison selon l'une quelconque des revendications précédentes, dans laquelle ladite croscarmellose sodique dans la phase extragranulaire à la croscarmellose sodique totale se situe dans la plage de 0,1 à 0,8, de préférence de 0,2 à 0,7 et, de façon davantage préférée, de 0,25 à 0,6.

16. Composition de combinaison selon l'une quelconque des revendications précédentes, comprenant les ingrédients suivants seulement :
a. couche à libération immédiate :
a. vildagliptine à raison de 3,5 à 60 % en poids ;
b. mannitol à raison de 5 à 90 % en poids ;
c. croscarmellose sodique à raison de 6 à 25 % en poids ;
d. polyvinylpyrrolidone à raison de 0,1 à 25 % en poids ;
e. dioxyde de silicium colloïdal à raison de 0,1 à 0,2 % en poids ;
f. stéarate de magnésium à raison de 0,25 à 2 % en poids ;
b. couche à libération contrôlée :
a. gliclazide à raison de 15 à 60 % en poids ;
b. phosphate de calcium dibasique à raison de 5 à 90 % en poids ;
c. éthyl cellulose/polyméthacrylate à raison de 0,5 à 40 % en poids ;
d. dioxyde de silicium colloïdal à raison de 0,1 à 0,2 % en poids ;
e. stéarate de magnésium à raison de 0,25 à 2 % en poids ;
c. couche de revêtement :
a. copolymère polyéthylène glycol-alcool polyvinylique ou polymère de méthacrylate à raison de 1 à 5 % en poids.

17. Procédé de préparation d'une formulation pharmaceutique selon l'une quelconque des revendications précédentes, comprenant les étapes de :
a. tamiser la vildagliptine, la croscarmellose sodique et le mannitol, et mélanger dans un granulateur à cisaillement élevé ;
b. granuler le mélange résultant avec une solution alcoolique/hydroalcoolique de polyvinylpyrrolidone ;
c. tamiser les granulés humides formés, sécher ceux-ci et tamiser les granulés séchés à nouveau ;
d. ajouter d'abord du dioxyde de silicium colloïdal, puis du stéarate de magnésium dans les granulés obtenus ;
e. tamiser du gliclazide et du phosphate de calcium dibasique, et mélanger dans un granulateur à cisaillement élevé ;
f. granuler le mélange résultant avec une solution hydroalcoolique d'éthyl cellulose/poly(méthacrylate de méthyle) ;
g. tamiser les granulés humides formés, sécher ceux-ci et tamiser les granulés séchés à nouveau ;
h. ajouter d'abord du dioxyde de silicium colloïdal puis du stéarate de magnésium dans les granulés obtenus ;
i. compacter la phase à libération contrôlée et la phase à libération immédiate pour fournir un comprimé bicouches ;
j. enrober le comprimé par un copolymère polyéthylène glycol-alcool polyvinylique ou un polymère de méthacrylate.

18. Composition de combinaison selon l'une quelconque des revendications précédentes pour prévenir ou traiter le diabète sucré dans des mammifères et, en particulier, dans des êtres humains.
